# EUROPÄISCHE PATENTANMELDUNG

(11) **EP 1 686 129 A1**
(43) Veröffentlichungstag der Anmeldung: **02.08.2006**
(21) Anmeldenummer: 04019643.8
(22) Anmeldetag: 18.08.2004
(51) Int. Cl.: C07D 477/20

(54) **Carbapenem-Verbindungen, ihre Herstellung und Verwendung in Synthese von Carbapenemen**

(71) Anmelder: SANDOZ AG, 4056 Basel (CH)
(72) Erfinder: Albert, Martin, 6262 Bruck am Ziller (AT); Kremminger, Peter, 6330 Kufstein (AT); Silberberger, Herbert, 6311 Oberau - Wildchönau (AT)
(74) Vertreter: Dietz, Jörg-Reimar

(57) **Zusammenfassung**

Die vorliegende Erfindung betrifft eine Verbindung der Formel V 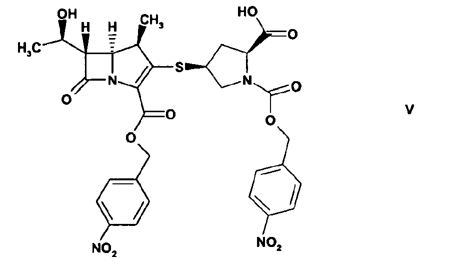 und dessen Salze sowie ein Verfahren zu dessen Herstellung. Desweiteren betrifft die Erfindung ein Verfahren zur Herstellung von 3-[*(3S*,*5S)*-5-carbamido-3-pyrrolidinyl]-thio substitutierten Carbapenemen wie Meropenem oder Ertapenem, worin eine Verbindung der Formel V oder dessen Salze als Zwischenprodukt verwendet werden.

## Beschreibung

Die vorliegende Erfindung betrifft organische Verbindungen, nämlich (*4R,5S,6S*)-(*p*-Nitrobenzyl) 3-[[(3S,5S)-1-(p-nitrobenzyloxycarbonyl)-5-carboxy-3-pyrrolidinyl]thio]-6-[(*1R*)-1-hydroxyethyl]-4-methyl-7-oxo-1-azabicyclo[3.2.0]hept-2-en-2-carboxylat und dessen Salze sowie Verfahren zur Herstellung von 3-[*(3S,5S)*-5-carbamido-3-pyrrolidinyl]-thio substitutierten Carbapenemen wie Meropenem oder Ertapenem, worin (4R,5S,6S)-(p-Nitrobenzyl) 3-[[(3S,5S)-1-(p-nitrobenzyloxycarbonyl)-5-carboxy-3-pyrrolidinyl]thio]-6-[(*1R*)-1-hydroxyethyl]-4-methyl-7-oxo-1-azabicyclo[3.2.0]hept-2-ene-2-carboxylat oder dessen Salze als Zwischenprodukt verwendet werden. 3-[*(3S,5S)*-5-carbamido-3-pyrrolidinyl]-thio substitutierte Carbapenem Verbindungen wie beispielsweise (4R,5S,6S)-3-[[(3S,5S)-5-[(Dimethylamino)carbonyl]-3-pyrrolidinyl]thio]-6-[(*1R*)-1-hydroxyethyl]-4-methyl-7-oxo-1-azabicyclo[3.2.0]hept-2-en-2-carbonsäure (INN: Meropenem) oder (4R,5S,6S)-3-[[(3S,5S)-5-[(3-carboxyphenyl)carbamoyl]-3-pyrrolidinyl]thio]-6-[(1R)-1-hydroxyethyl]-4-methyl-7-oxo-1-azabicyclo[3.2.0]hept-2-en-2-carbonsäure (INN: Ertapenem) besitzen hervorragende antibakterielle Eigenschaften gegenüber einem breiten Spektrum an sowohl Gram-negativen wie Gram-positiven Keimen (siehe z.B. The Merck Index 13th Edition, 2001, Item 5926 und Item 3706).

Bei bekannten Verfahren zur Herstellung von 3-[*(3S,5S)*-5-carbamido-3-pyrrolidinyl]-thio substitutierten Carbapenemen ist es erforderlich, eine Vielzahl an Zwischenstufen aufzureinigen und zu isolieren, um die Carbapeneme in ausreichender Reinheit und Ausbeute zu erhalten. Es ist beispielsweise bekannt, dass die Herstellung von Meropenem der Formel 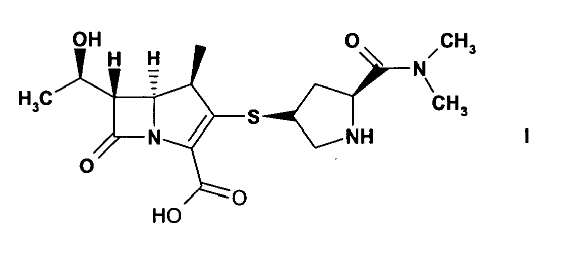 etwa dadurch erfolgen kann, dass die Mercapto-Seitenkette (*2S,4S*)-2-Dimethylaminocarbonyl-4-mercapto-pyrrolidin in der Form eines Salzes über eine Additionsreaktion in die 3-Position des Carbapenemgerüsts eingeführt werden kann, wobei sowohl die Carboxylgruppe des Carbapenemgerüsts als auch die sekundäre Aminogruppe des Pyrrolidinkörpers in geeigneter Weise, z.B. als *para*-Nitrobenzylester bzw. *para-*Nitrobenzylcarbamat, geschützt, vorliegen können. Durch Abspaltung dieser Schutzgruppen, z.B. durch Hydrierung, wird dann Meropenem der Formel I erhalten.
Nachteilig bei der Durchführung dieser Synthese ist, dass die für die 3-Position vorgesehene geschützte Seitenkette (*2S,4S*)-2-Dimethylaminocarbonyl-4-mercapto-1-(*p-*nitrobenzyloxycarbonyl)-pyrrolidin über eine vielstufige Synthese hergestellt werden muß, in welcher beispielsweise im letzten Schritt das bicyclische Thiolacton (*1S,4S*)-5-(4-Nitrobenzyloxycarbonyl)-2-thia-5-azabicyclo[2.2.1]heptan-3-on mit Dimethylamin geöffnet wird (siehe beispielsweise Heterocycles, Vol 41, No.1, 1995, 147-159 oder Tetrahedron Letters, Vol. 37, No. 17, pp. 2919-2922), um die gewünschte Seitenkette in der Form des Dimethylamids zu erhalten.

Es wurde nun überraschenderweise ein Verfahren zur Herstellung von 3-[(*3S,5S*)-5-carbamido-3-pyrrolidinyl]-thio substitutierten Carbapenemen, wie z.B. Meropenem oder Ertapenem, gefunden, bei dem eine neue Zwischenverbindung gebildet wird, so dass das bicyclische Thiolacton (*1S,4S*)-5-(4-Nitrobenzyloxycarbonyl)-2-thia-5-azabicyclo[2.2.1]heptan-3-on direkt, d.h. ohne isoliert zu werden, eingesetzt werden kann.

Daher betrifft die vorliegende Erfindung in einem Aspekt ein Verfahren zur Herstellung von 3-[*(3S,5S)*-5-carbamido-3-pyrrolidinyl]-thio substitutierten Carbapenemen der Formel 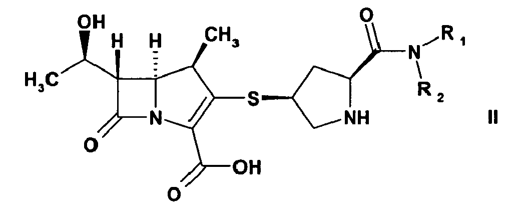 worin R₁ und R₂ unabhängig voneinander Alkyl, Wasserstoff, Aryl oder Heterocyclyl bedeutet, oder R₁ und R₂ zusammen mit dem Stickstoffatom der Amid-Gruppe einen 5- bis 8 gliedrigen gesättigten oder ungesättigten Heterocyclus bilden, wobei das Verfahren folgende Schritte umfasst:
(A) Reaktion einer Verbindung der Formel 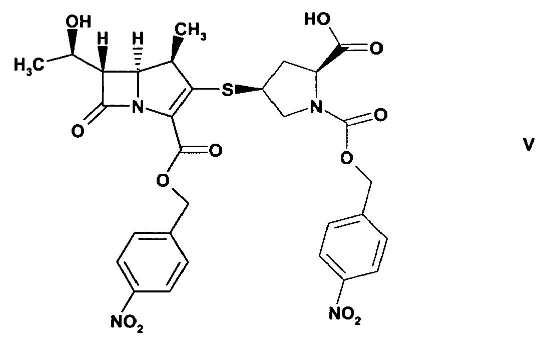 worin die Carboxylgruppe an der 5-Position des Pyrolidinylthio-Rests in freier Form, in der Form eines Salzes oder in aktivierter Form vorliegen kann, mit einem Amin der Formel 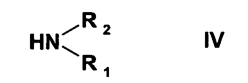 worin R₁ und R₂ dieselben Bedeutungen wie in Formel II haben, um eine Verbindung der Formel 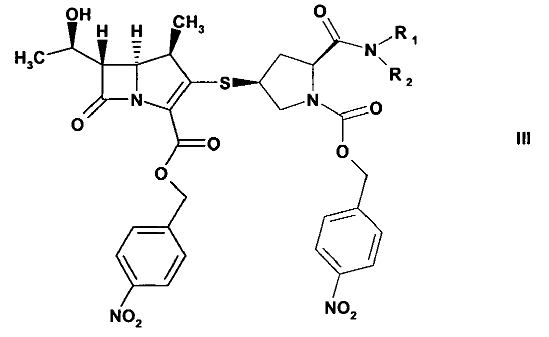 zu erhalten, worin R₁ und R₂ dieselben Bedeutungen haben wie oben für Formel II definiert,
(B) Abspaltung der p-Nitrobenzyl- bzw. p-Nitrobenzylcarbonyl-Schutzgruppen aus einer Verbindung der Formel III wie aus Schritt A erhalten, um eine Verbindung der Formel II zu erhalten.

Wenn nichts anderes vermerkt ist, so schliesst Alkyl im Rahmen dieser Erfindung verzweigtes oder unverzweigtes (C₁₋₈)Alkyl ein, wie (C₁₋₄)Alkyl, z.B. Methyl, Ehtyl, iso-Propyl, n-Propyl. Alkyl kann unsubstituiert sein oder aber einfach oder mehrfach, z.B. zweifach oder dreifach, substituiert sein. Als mögliche Substituenten kommen beispielsweise in Frage Hydroxy, Amino, Alkyl-Amino, Dialkyl-Amino, Aryl oder Heterocyclyl. Cycloalkyl schliesst, wenn nichts anderes angegeben ist, im Rahmen dieser Erfindung (C₃₋₆)Cycloalkyl ein wie z.B. Cyclopropyl, Cyclopentyl oder Cyclohexyl, insbesondere Cyclohexyl. Cycloalkyl kann unsubstituiert sein oder substituiert mit z.B. Alkyl, Hydroxy, Amino, Alkyl-Amino, Dialkyl-Amino, Aryl oder Heterocyclyl.
Alkoxy ist insbesondere (C₁₋₈)Alkoxy wie (C₁₋₄)Alkoxy, z.B. Methoxy.
Sofern nichts anderes angegeben ist, so bedeutet Heterocyclyl im Rahmen dieser Erfindung einen 5- bis 8-gliedrigen, vorzugsweise 5- bis 6-gliedrigen, gesättigten oder ungesättigten Heterocyclus mit 1 bis 4, z.B. 1 bis 3 Heteroatomen ausgewählt aus der Gruppe bestehend aus N, S und O, bevorzugt N. Heterocyclyl schliesst unsubstituierte Heterocyclen wie auch substituierte Heterocyclen ein. Als Substituenten für Heterocyclyl kommen beispielsweise in Frage Alkyl, Carboxy, Hydroxy, Amino, Alkylamino, Dialkylamino, (C₁₋₈)-Alkoxy, Oxo, Thioxo. Beispiele für Heterocyclyl sind Pyridinyl, Pyrazolyl, Pyridyl, Pyrazinyl, Furyl, Thienyl, Imidazolyl, Thioxazolyl, Morpholinyl oder Triazolyl. Aryl ist bevorzugt unsubstituiertes oder ein oder mehrfach, z.B. 1 bis 2 fach substitutiertes Phenyl. Als Substituenten für Aryl kommen beispielsweise in Frage Alkyl, Carboxy, Alkoxycarbonyl, Phenylmethoxycarbonyl, Hydroxy, Nitro, Amino, Alkylamino, Dialkylamino, Alkoxy. Ist eine Aryl-Gruppe mit einer Carboxygruppe substituiert so kann diese Carbxygruppe gegebenenfalls geschützt sein. Als Schutzgruppen kommen bekannte übliche Carboxy-Schutzgruppen in Frage wie z.B. Alkyl, Aryl oder Aralkylgruppen. Daher kann Aryl beispielsweise mit einer oder meheren Phenylmethoxycarbonylgruppen substituiert sein, worin die letztere Phenylgruppe ihrerseits unsubstituiert oder mit einem der für Aryl oben erwähnten Substituenten substituiert sein kann, insbesondere mit 1 bis 5, z.B. einer oder zwei Nitrogruppen. Bevorzugte Substituenten für Aryl sind beispielsweise 4-Nitro-Phenylmethoxycarbonyl, 3-Carboxy, Alkoxy-carbonylphenyl oder Phenylmethoxycarbonyl.

In einer bevorzugten Ausführungsform stehen R₁ und R₂ beide für Methyl.
In einer anderen bevorzugten Ausführungsform bedeutet R₁ Wasserstoff und R₂ *meta-*Carboxyphenyl, dessen Carboxy-Gruppe frei, als Salz oder mit einer Schutzgruppe geschützt vorliegen kann. Ist die Carboxygruppe geschützt, so bedeutet R₂ insbesondere 3-(4-Nitro-Phenylmethoxycarbonyl)phenyl, 3-(Alkoxy-carbonylphenyl) oder 3-(Phenylmethoxycarbonyl)-phenyl.

In einer bevorzugten Ausführungsform wird in Schritt A die Verbindung der Formel V in einer Form eingesetzt, bei der die Carboxylgruppe an der 5-Position des Pyrolidinylthio-Rests aktiviert wurde.
Geeignete Methoden der Aktivierung sind dem Fachmann bekannt. Bevorzugte Methoden der Aktivierung sind die Bildung eines Aktivesters, vorzugsweise des Mercaptobenzothiazolylesters, durch Reaktion mit Bis-(benzothiazol-2-yl)-disulfid in Gegenwart eines Phosphins, beispielsweise Triphenylphosphin, oder eines Phosphits, beispielsweise Triethylphosphit. Weitere bevorzugte Methoden der Aktivierung schliessen die Bildung eines gemischten Anhydrids bei Temperaturen zwischen -60°C und +10°C, bevorzugt zwischen -30°C und 0°C, in Gegenwart einer Base, oder die Bildung eines Carbonsäurechlorids, eventuell in Gegenwart eines geeigneten Katalysators. Besonders bevorzugte Methoden zur Aktivierung sind die Umsetzung mit einem Phosphinsäurechlorid wie z.B. Diphenylphosphinsäurechlorid, mit einem Phosphorsäurechlorid wie Chlorphosphorsäure-diphenylester oder einem Carbonsäurechlorid wie Pivaloylchlorid. Bevorzugte Basen sind sekundäre oder tertiäre Amine, ein Amidin oder Guanidin. Besonders bevorzugte Amine sind Triethylamin, Diisopropylethylamin und Diisopropylamin, besonders bevorzugte Amidine sind 1,8-Diazabicyclo[5.4.0]undec-7-en (DBU) und 1,5-Diazabicyclo[4.3.0]non-5-en (DBN) und besonders bevorzugtes Guanidin ist Tetramethylguanidin. Als Katalysatoren verwendet man vorzugsweise Pyridin, Dimethylaminopyridin, Picolin, Lutidin oder Collidin.
Die Umsetzung einer gegebenenfalls aktivierten Carbonsäure der Formel V mit einem Amin der Formel IV in Schritt (A) wird analog zu den für Amidbildungsreaktionen üblichen Bedingungen durchgeführt, vorzugsweise in einem aprotischen Lösungsmittel bei Temperaturen zwischen -50°C und +10°C. Geeignete Lösungsmittel sind Ester wie Ethylacetat oder Butylacetat, Ether wie Tetrahydrofuran oder Dioxan, Nitrile wie Acetonitril, halogenierte aliphatische Kohlenwasserstoffe wie Dichlormethan, halogenierte oder nicht halogenierte aromatische Kohlenwasserstoffe wie Chlorbenzol oder Toluol, Amide wie Dimethylformamid oder Dimethylacetamid, Sulfoxide wie Dimethylsulfoxid und Ketone wie Aceton oder Methylisopropylketon, sowie Mischungen dieser Lösungsmittel. Besonders bevorzugt sind Dimethylformamid, Dimethylacetamid, Ethylacetat und Methylenchlorid sowie Mischungen derselben. Bevorzugter Temperaturbereich ist -30°C bis +10°C. Anstatt des Amins der Formel IV kann auch ein geeignetes Salz des Amins, beispielsweise ein Hydrochlorid, in Gegenwart einer Base oder ein geeignetes Derivat eines Amins, das ein Amin der Formel IV freisetzen kann, eingesetzt werden.

Derivate von Aminen der Formel IV schliesst z.B. Dimethylcarbamate der Amine, z.B. Dimethylammonium-dimethylcarbamat, oder Trialkylsilyl substituierte Amine, wie z.B. N,N-Dimethyl-(trimethylsilyl)amin, ein.
Nach der Umsetzung mit dem Amin der Formel IV wird mit Wasser oder wässrigen Lösungen anorganischer Salze, vorzugsweise Natriumchlorid, extrahiert und das Reaktionsprodukt der Formel III in reiner Form aus der organischen Phase isoliert. Die Abspaltung der Schutzgruppen in Schritt (B) des erfindungsgemässen Verfahrens kann analog, z.B. gemäss bekannten Methoden erfolgen. Die Abspaltung der Schutzgruppen kann beispielsweise durch Hydrolyse mit geeigneten anorganischen oder organischen Säuren geschehen. Eine weitere Variante ist die katalytische Hydrierung gemäss bekannten Verfahren. Enthält R₂ eine geschützte Carboxygruppe so kann jene Schutzgruppe gleichzeitig mit den p-Nitrobenzyl- bzw. p-Nitrobenzylcarbonyl-Schutzgruppender Formel III unter geeigneten, bekannten Bedingungen abgespalten werden, um die freie Carboxygruppe, gegebenenfalls in der Form eines Salzes, zu erhalten.

Eine Verbindung der Formel V wie sie als Ausgangsmaterial in Schritt (A) des oben beschriebenen Verfahrens vorgesehen ist, ist neu und deswegen ist ein weiterer Aspekt der vorliegenden Erfindung eine Verbindung der Formel 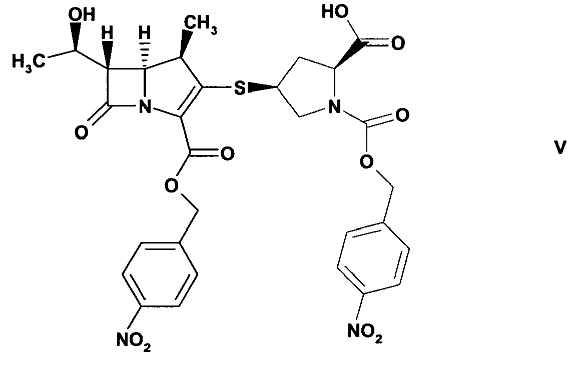 in freier Form oder in Form eines Salzes, z.B. mit einem geeigneten Kation an der freien Carboxylat-Gruppe. Geeignete Kationen sind beispielsweise Metall-, Ammonium-Kationen oder Kationen von organischen Basen wie primäre, sekundäre oder tertiäre Amine. Bevorzugte Salze der Verbindung der Formel V sind die Salze mit Ammonium-, Erdalkalimetall- oder Alkalimetallkationen, insbesondere Lithium, Natrium, Kalium, Ammonium, Magnesium, Calcium, wie z.B. Natrium, Kalium, Lithium oder Ammonium. Die Umwandlung einer Verbindung der Formel V in freier Form in eine Verbindung der Formel V in Salzform und umgekehrt kann analog zu bekannten Methoden erfolgen, z.B. durch Reaktion der Verbindung V in Form der freien Säure mit einer Quelle von geeigneten Kationen. Verbindungen der Formel V sind nützliche Zwischenprodukte bei der Herstellung von Carbapenemen der Formel II. Deshalb ist ein weiterer Aspekt der vorliegenden Erfindung die Verwendung einer Verbindung der Formel V bei der Herstellung von 3-[(*3S,5S*)-5-carbamido-3-pyrrolidinyl]-thio substituierten Carbapenemen der Formel II wie oben definiert, bevorzugt bei der Herstellung von Ertapenem oder Meropenem, insbesondere von Meropenem.

Desweiteren betrifft die vorliegende Erfindung ein Verfahren zur Herstellung von Verbindungen der Formel V umfassend die Schritte
(i) Hydrolyse einer Verbindung der Formel 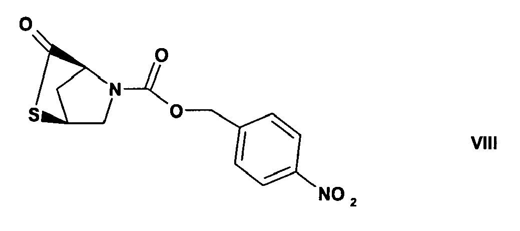 um eine Verbindung der Formel 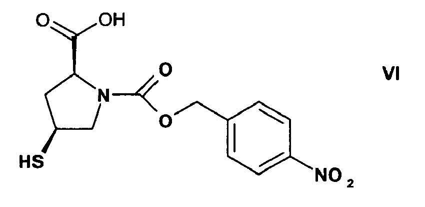 zu erhalten, und
(ii) Reaktion einer Verbindung der Formel 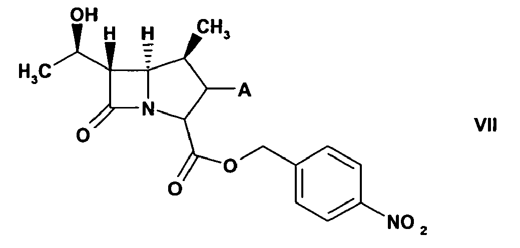
worin A für eine Aktivierungsgruppe steht, mit einer Verbindung der Formel VI wie aus Schritt (i) erhalten, um eine Verbindung der Formel V zu erhalten.

Eine Aktivierungsgruppe A ist eine funktionelle Gruppe, die die Reaktivität des CarbapenemGerüsts an der 3-Position steigert. Eine Aktivierungsgruppe umfasst insbesondere reaktive Ester einer Carbapenem Verbindung, die an der 3-Position mit einer Hyroxygruppe substituiert ist. Eine Aktivierungsgruppe umfasst insbesondere ebenfalls eine substituierte oder unsubstituierte Alkyl-Sulfinylgruppe. Bevorzugt ist eine di-Aryl-Phosphorsäureester Gruppe wie z.B. Di-Phenylphosphorsäure. Daher steht A bevorzugt für eine Gruppe der Formel 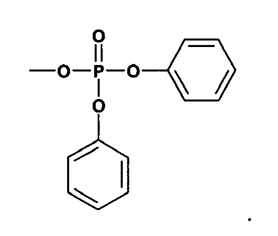 Schritt (i) wird unter den für Hydrolysereaktionen von Lactonen oder Thiolactonen üblichen Bedingungen durchgeführt, vorzugsweise unter basischer Katalyse in Gegenwart eines organischen Co-Lösungsmittels oder in rein organischen Lösungsmitteln. Typische Temperaturbereiche sind -30°C bis +30°C, vorzugsweise -10°C bis +10°C. Die ringoffene Carbonsäure der Formel VI wird durch Ansäuern erhalten und kann gegebenenfalls durch Extraktion mit einem mit Wasser nicht oder nur teilweise mischbaren organischen Lösungsmittel in reiner gelöster Form erhalten werden. In einer bevorzugten Ausführungsform kann die Lösung der ringoffenen Carbonsäure der Formel VI *in situ* direkt im Schritt (ii) eingesetzt werden. Als Lösungsmittel für diese Extraktion sind geeignet z.B. Ester, chlorierte Kohlenwasserstoffe, Ketone oder aromatische Kohlenwasserstoffe. Bevorzugte Lösungsmittel sind Dichlormethan, Ethylacetat, Propylacetat, Butylacetat, Methylisobutylketon oder Toluol, besonders bevorzugt sind Dichlormethan und Ethylacetat.

Schritt (ii) der Reaktion wird unter den für eine Michael-Addition typischen bekannten Bedingungen durchgeführt, in Gegenwart einer Base, vorzugsweise ein sekundäres oder tertiäres Amin, ein Amidin oder Guanidin. Besonders bevorzugte Amine sind Triethylamin, Diisopropylethylamin und Diisopropylamin. Besonders bevorzugte Amidine sind 1,8-Diazabicyclo[5.4.0]undec-7-en (DBU) und 1,5-Diazabicyclo[4.3.0]non-5-en (DBN). Ein besonders bevorzugtes Guanidin ist Tetramethylguanidin. Schritt (ii) der Reaktion läuft in einem Temperaturbereich zwischen -60°C und +10°C ab, bevorzugt zwischen -30°C und -10°C. Als Lösungsmittel eignen sich nicht-protische Lösungsmittel, beispielsweise Ester, z.B. Ethylacetat oder Butylacetat, Ether wie Tetrahydrofuran oder Dioxan, Nitrile wie Acetonitril, halogenierte aliphatische Kohlenwasserstoffe wie Dichlormethan, halogenierte und nicht halogenierte aromatische Kohlenwasserstoffe wie Chlorbenzol oder Toluol, Amide wie Dimethylformamid oder Dimethylacetamid, Sulfoxide wie Dimethylsulfoxid und Ketone wie Aceton oder Methylisopropylketon. Nach erfolgter Umsetzung wird angesäuert, mit Wasser versetzt und durch Extraktion mit einem mit Wasser nicht mischbaren Lösungsmittel von den Salzen abgetrennt. Eine Verbindung der Formel V als Produkt einer solchen Reaktion wird so in reiner Form als Lösung in einem organischen Lösungsmittel erhalten und eignet sich ohne Zwischenisolierung für die direkte weitere Umsetzung, z.B. in Schritt (A) eines Verfahrens zur Herstellung einer Verbindung der Formel II, wie oben beschrieben.

Wenn gewünscht, kann die gebildete Verbindung der Formel V in einer alternativen Ausführungsform auch aus der Reaktionsmischung isoliert werden, z.B. in Form eines Salzes. Dazu wird die Lösung nach einer wässrigen Extraktion entweder mit einem Antilösungsmittel, das die Löslichkeit einer Verbindung der Formel V herabsetzt wie vorzugsweise einem Ether oder einem Kohlenwasserstoff, versetzt oder es wird zur Salzbildung mit Alkalimetallsalzen anderer Carbonsäuren, beispielsweise mit Natrium-, Kalium- oder Lithiumethylhexanoat oder Natrium-, Kalium- oder Litiumacetat versetzt. Durch die Zugabe von salzbildenden Reagentien fällt das Kupplungsprodukt der Formel V als Salz, vorzugsweise in kristalliner Form, aus.

Die Ausgangsverbindungen der Formeln VIII und VII können gemäss bekannten Verfahren hergestellt werden.

Ein besonderer Vorteil der hier beschriebenen erfindungsgemässen neuen Verfahren liegt darin, dass die gesamte Sequenz bis zu den Carbapenem-Verbindungen der Formel II, wie Meropenem oder Ertapenem, ohne Zwischenisolierung durchgeführt werden kann. Die Reinigung der Zwischenstufen sowie die Abtrennung der bei den einzelnen Reaktionsschritten angefallenen Salze wird nur über Extraktionsschritte durchgeführt, was eine Vereinfachung gegenüber aufwändigen Kristallisationsaufreinigungsschritten darstellt, die meist immer mit einem Ausbeuteverlust einhergehen.

So kann in einer besonderen Ausführungsform der vorliegenden Erfindung (*1S,4S*)-5-(4-Nitrobenzyloxycarbonyl)-2-thia-5-azabicyclo[2.2.1]heptan-3-on *in situ* zu der entsprechenden 4-Mercaptopyrrolidin-2-carbonsäure hydrolysiert werden, die direkt mit dem Carbapenemnucleus umgesetzt und in Folge ohne Zwischenisolierung mit einem Amin der Formel IV zum entsprechenden Amid weiterverarbeitet werden kann, aus welchem ein 3-[*(3S,5S)*-5-carbamido-3-pyrrolidinyl]-thio substitutiertes Carbapenem, z.B. Meropenem oder Ertapenem in einer für pharmazeutische Anwendungen geeigneten Form erhalten werden kann.

Das nach den oben dargelegten erfindungsgemässen Verfahren hergestellte Carbapenem der Formel II, z.B. Meropenem oder Ertapenem, kann als Wirkstoff in einer pharmazeutischen Zusammensetzung eingesetzt werden. Ein letzter Aspekt dieser Erfindung betrifft daher ein Verfahren zur Herstellung einer pharmazeutischen Zusammensetzung enthaltend ein Carbapenem der Formel II, umfassend die Schritte A und B, sowie optional zusätzlich die Schritte (i) und (ii) der oben beschriebenen Verfahren und des weiteren umfassend das Mischen des aus Schritt B erhaltenen Verbindung der Formel II mit Lösungsmitteln oder Hilfsstoffen, um eine pharmazeutische Zusammensetzung zu erhalten.
Die folgenden Beispiele sollen die vorliegende Erfindung veranschaulichen und sind keinesfalls als limitierend für den Schutzumfang zu verstehen. Alle Temperaturen sind in °Celsius, und alle Reinheiten in HPLC-Flächenprozenten angegeben.

### Beispiele

### Beispiel 1: a) (4R,5S,6S)-(p-Nitrobenzyl) 3-[[(3S,5S)-1(p-nitrobenzyloxycarbonyl)-5-carboxy-3-pyrrolidinyl]thio]-6-[(1R)-1-hydroxyethyl]-4-methyl-7-oxo-1-azabicyclo[3.2.0]hept-2-ene-2-carboxylat

5,0g (*1S,4S*)-5-(4-Nitrobenzyloxycarbonyl)-2-thia-5-azabicyclo[2.2.1]heptan-3-on werden bei 0°C in 1000ml Acetonitril gelöst und es werden 270mg Natriumdithionit in 2000ml Wasser rasch zugegeben. Durch Zugabe von 1 N NaOH wird der pH-Wert auf 12,8 eingestellt und 60min nachgerührt. Dann wird mit 2N HCl auf pH 2,3 gestellt und es wird mit 200ml Dichlormethan versetzt. Die obere organische Phase wird abgetrennt, mit Wasser gewaschen und im Vakuum eingeengt. Erhalten werden 6,5g gelbes Öl mit einer HPLC-Reinheit von >99 %.
Der erhaltene Eindampfrückstand wird in 90ml Dimethylacetamid gelöst und auf -25°C temperiert. Nach der Zugabe von 9,2g (*4R,5S,6S*)-(*p*-Nitrobenzyl) 3-[(diphenylphosphono)oxy]-6-[(*1R*)-1-hydroxyethyl]-4-methyl-7-oxo-1-azabicyclo[3.2.0]hept-2-ene-2-carboxylat sowie 4,3ml Tetramethylguanidin wird noch 100min nachgerührt. Nach beendeter Umsetzung werden 2,4ml Essigsäure und 100ml Ethylacetat zudosiert und es wird mit 100ml einer 10%igen NaCl Lösung extrahiert. Die wässrige Phase wird mit 50ml Ethylacetat gewaschen und die vereinigten organischen Phasen werden dreimal mit je 50ml 10%iger NaCl-Lösung gewaschen. Die Ethylacetat Phase wird über Na₂SO₄ getrocknet. Nach dem Abfiltrieren des Trockenmittels wird die Ethylacetat-Phase auf 3000ml Diisopropylether getropft, wobei das gewünschte Produkt ausfällt. Es wird noch 10 min nachgerührt, abfiltriert, mit Diisopropylether gewaschen und getrocknet.
Auswaage: 10,3g
¹H-nmr (DMSO-d₆): δ 12.9(br s,1H), 8.21 (m,4H), 7.72(d,J=9.0Hz,2H), 7.65&7.60(2d,J=9.0Hz,2H), 5.46&5.30(ABq,J=13.8Hz, 2H), 5.3-5.14(m,2H), 5.09(br s,1H), 4.5-4.22(m,2H), 4.22-4.05(m,1H), 4.05-3.85(m,2H), 3.55(m,1H), 3.27(m,2H), 2.84(m,1 H), 1.88(m,1H), 1.17(2d,J=6.0Hz,6H)
¹³C-nmr (DMSO-d₆): δ 174.38, 174.25, 173.23, 172.77, 160.12, 153.47, 153.18, 150.58, 147.27, 147.18, 147.12, 144.87, 144.77, 143.92, 128.52, 128.30, 128.01, 124.01, 123.91, 123.72, 123.63, 65.29, 65.04, 64.33, 59.89, 58.83, 58.29, 55.58, 43.26, 36.16, 35.31, 21.91, 17.24

### b) (4R,5S,6S)-(p-Nitrobenzyl) 3-[[(3S,5S)-1 (-p-nitrobenzyloxycarbonyl)-5-(dimethylaminocarbonyl)-3-pyrrolidinyl]thio]-6-[(1R)-1-hydroxyethyl]-4-methyl-7-oxo-1-azabicyclo[3.2.0]hept-2-ene-2-carboxylat

4,05g des aus Beispiel 1a) erhaltenen Materials werden in 50ml Dimethylacetamid gelöst und bei -20°C werden 1,33ml Diisopropylethylamin, gefolgt von 1,20ml Diphenylphosphinsäurechlorid zugegeben. Es wird 15 min nachgerührt und die Lösung wird bei -10°C zu einer Lösung von 0,81g Dimethylamin in 30ml Dimethylacetamid zugetropft. Nach 1 h werden 50ml Ethylacetat und 30ml 10%ige NaCl-Lösung zugegeben und die Phasen getrennt. Die wässrige Phase wird mit 30ml Ethylacetat gewaschen und die vereinigten Ethylacetat-Phasen werden dreimal mit je 15ml 10%iger NaCl Lösung gewaschen. Die Ethylacetat-Phase wird im Vakuum eingeengt, angeimpft und bei 0°C zur Kristallisation stehen gelassen. Die Kristalle werden filtriert, mit kaltem Ethylacetat gewaschen und im Vakuum getrocknet.
Auswaage: 3,76g
¹H-NMR (DMSO-d₆) δ 8.24 (m, 4H), 7.73 (d, J = 9.00 , 2H), 7.66&7.54 (d, J = 9.0, 2H), 5.47&5.30(ABq, 2H,J=14.1 Hz), 5.25-5.04(m,3H), 4.80(m,1 H), 4.26(m,1H), 4.15(m,1 H), 3.99(m,1 H), 3.86(m,1 H), 3.61(m,1H), 3.29(m,1 H), 3.20(m,1H), 3.03&2.97(2s,3H), 2.87(m,1H), 2.83&2.83(2s,3H), 1.63(m,1H), 1.17(2t, 6H,J=6.0Hz)
¹³C-NMR (DMSO-d₆) δ 174.39, 174.27, 170.85, 170.49, 160.09, 153.10, 152.83, 150.51, 150.44, 147.24, 147.12, 147.10, 144.85, 143.89, 128.47, 128.21, 127.94, 123.93, 123.81, 123.66, 123.57, 65.09, 65.06, 64.29, 64.23, 59.97, 59.90, 56.39, 56.04, 55.49, 55.00, 54.40, 43.17, 36.52, 36.44, 35.90, 35.49, 35.18, 21.90, 21.85
Schmelzpunkt: 156-159°C
IR (Golden gate) cm⁻¹: 1770, 1710, 1640, 1525, 1340, 1210, 1140, 850, 740

### Beispiel 2: a) (4R,5S,6S)-(p-Nitrobenzyl) 3-[[(3S,5S)-1(-p-nitrobenzyloxycarbonyl)-5-(lithium carboxylato)-3-pyrrolidinyl]thio]-6-[(1R)-1-hydroxyethyl]-4-methyl-7-oxo-1-azabicyclo[3.2.0]hept-2-ene-2-carboxylat

5.0 g [16.2 mmol] (*2S,4S*)-4-Mercapto-pyrrolidin-1,2-dicarbonsäure-1-(4-nitro-benzyl)ester werden in 100 ml THF oder Acetonitril gelöst. Bei 10°C werden innerhalb von 30 Minuten 2 equiv. NaOH in Form einer 1 M wässrigen NaOH-Lösung (32.4 ml) zugegeben. Nach erfolgter Zugabe wird 10 Minuten gerührt. Anschließend werden 480 µl Tributylphosphin zugegeben. Es wird weitere 5 Minuten gerührt. Nach Zugabe von 50 ml Essigester und 20 ml H₂O werden die Phasen getrennt und die wässrige Phase wird nach Zugabe von 50 ml CH₂Cl₂ mit 2 N wässriger HCl (32 ml) auf einen pH-Wert von 2.5 gestellt. Nach 5 Minuten Rühren bei Raumtemperatur werden die Phasen getrennt und die organische Phase über Na₂SO₄ getrocknet. Nach Filtration werden 30 ml N,N-Dimethylacetamid zugegeben. CH₂Cl₂ und Acetonitril/THF werden unter vermindertem Druck (Badtemperatur 35°C, 20mbar) abgezogen. Die Lösung von (*2S,4S*)-1-(p-Nitrobenzyloxycarbonyl)-4-Mercapto-pyrrolidin-2-carbonsäure in N,N-Dimethylacetamid wird direkt für den nächsten Schritt eingesetzt. Zur Lösung von (*2S,4S*)-1-(p-Nitrobenzyloxycarbonyl)-4-Mercapto-pyrrolidin-2-carbonsäure in N,N-Dimethylacetamid werden weitere 80 ml N,N-Dimethylacetamid zugegeben. Bei -25°C erfolgt die Zugabe von 8.80 g [14.8 mmol] (*4R,5S,6S*)*-*(*p*-Nitrobenzyl) 3-[(diphenylphosphono)oxy]-6-[(*1R*)*-*1-hydroxyethyl]-4-methyl-7-oxo-1-azabicyclo[3.2.0]hept-2-ene-2-carboxylat. Zu dieser leicht gelben Lösung werden nun innerhalb von 30 Minuten 4.26 mL Tetramethylguanidin [33.9 mmol] in 5 ml *N,N*-Dimethylacetamid zugetropft. Bei der Zugabe sollte die Innentemperatur -20°C nicht übersteigen. Es wird 30 Minuten bei -25°C nachgerührt. Danach wird die Reaktionslösung innerhalb von 35 Minuten auf ein auf -5°C vorgekühltes Gemisch von 100 ml H₂O, 100 ml Essigester (Ethylacetat) und 4 ml H₃PO₄ (85%ig) getropft. Das zweiphasige Gemisch wird 5 Minuten nachgerührt. Die untere wässrige Phase wird abgelassen und erneut bei 0°C mit 15 ml Essigester extrahiert. Die vereinten Essigester-Phasen werden einmal mit gesättigter NaCl-Lösung gewaschen (bei 0°C) und anschließend über Na₂SO₄ getrocknet. Nach Filtration werden rund 100 g organische Phase erhalten.
Bei 0°C werden zur Essigester-Lösung 2.1 g [14.1 mmol] Lithium 2-Ethylhexanoat zugegeben. Ein feiner kristalliner Niederschlag entsteht bereits bei der Zugabe. Bei 0°C wird ein halbe Stunde nachgerührt. Die Kristallsuspension wird weitere 15h bei 4°C gelagert und danach filtriert. Es werden nach Trocknen bei 25°C unter vermindertem Druck (20 mbar) 8.7 g an (*4R,5S,6S*)-(*p*-Nitrobenzyl) 3-[[(*3S,5S*)-1(-p-nitrobenzyloxycarbonyl)-5-(lithium carboxylato)-3-pyrrolidinyl]thio]-6-[(*1R*)-1-hydroxyethyl]-4-methyl-7-oxo-1-azabicyclo[3.2.0]hept-2-ene-2-carboxylat erhalten.
Schmelzpunkt: 220-222°C Zersetzung
¹H-NMR (DMSO-d₆): δ 8.30-8.10 (m, 4H), 7.80-7.60 (m, 4H), 5.5-5.0 (m, 5H), 4.24 (d, J 7.2 Hz, 1 H), 4.15-3.90 (m, 3H), 3.73 (m, 1H), 3.56 (m, 1 H), 3.3-3.1 (m, 2H), 2.68 (m, 1 H), 1.76 (m, 1H), 1.19-1.16 (6H)
¹³C-NMR (DMSO-d₆): δ 174.2, 174.0, 173.7, 173.3, 160.0, 153.5, 153.0, 151.6, 147.1, 146.8, 146.6, 145.7, 145.3, 143.8, 128.3, 127.9, 127.5, 123.6, 123.5, 123.3, 123.2, 65.6, 65.2, 65.0, 62.0, 61.7, 60.6, 56.3, 55.5, 44.0, 37.6, 36.8, 21.7, 17.2
IR(Golden Gate) 1767, 1704, 1606, 1519, 1414, 1342, 1289, 1209, 1166, 1109, 1047, 853, 803, 763, 736, 694

### b) (4R,5S,6S)-(p-Nitrobenzyl) 3-[[(3S,5S)-1(-p-nitrobenzyloxycarbonyl)-5-(dimethylaminocarbonyl)-3-pyrrolidinyl]thio]-6-[(1R)-1-hydroxyethyl]-4-methyl-7-oxo-1-azabicyclo[3.2.0]hept-2-ene-2-carboxylat

1 g [1.48 mmol] des Produkts aus Stufe 2a) werden in 20 ml Dimethylformamid gelöst. 80µl [0.46 mmol] Diisopropylethylamin werden bei -30°C zugegeben. Bei derselben Temperatur werden 230 µl [1.86 mmol] Pivaloylchlorid zur Lösung zugetropft. Die Temperatur soll -20°C nicht überschreiten. Nach vollständiger Bildung des gemischtes Anhydrids wird langsam eine Suspension von 370 mg [4.53 mmol] Dimethylammonium chlorid und 776 µl Diisopropylethylamin [4.53 mmol] in 10 ml DMF zur Lösung zugegeben. Nach 90 Minuten bei -20°C werden 50 ml Essigester und 25 ml 5%ige wässrige NaCl zugegeben. Es wird 5 Minuten nachgerührt. Nach Phasentrennung wird die organische Phase nochmals mit 10 ml 5%iger wässriger NaCl nachgewaschen. Bei -10°C wird die Essigester-Phase nun einmal mit 15 ml gesättigter NaCl-Lösung gewaschen. Nach Phasentrennung wird die organische Phase über Na₂SO₄ getrocknet. Die Essigester-Phase wird unter vermindertem Druck eingedampft bis ein erste Trübung zu beobachten ist. 30 Minuten wird bei 25°C gerührt, anschließend 15h bei 4°C gelagert. Die Kristalle werden daraufhin abfiltriert und getrocknet. (Ausbeute: 0.92g)

Die physikalischen und spektroskopischen Daten sind identisch wie bei dem Produkt aus Beispiel 1b).

### Beispiel 3: (4R,5S,6S)-(p-Nitrobenzyl) 3-[[(3S,5S)-1(-p-nitrobenzyloxycarbonyl)-5-(dimethylaminocarbonyl)-3-pyrrolidinyl]thiol-6-[(1R)-1-hydroxyethyl]-4-methyl-7-oxo-1-azabicyclo[3.2.0]hept-2-ene-2-carboxylat

5,0g (*1S,4S*)-5-(4-Nitrobenzyloxycarbonyl)-2-thia-5-azabicyclo[2.2.1]heptan-3-on werden bei 5°C in 100ml Acetonitril gelöst und es wird eine Lösung von 280mg Natriumdithionit in 200ml Wasser zugegeben. Unter Kühlung auf -6°C wird unter langsamer Zugabe von 48ml 1M Natronlauge der pH-Wert auf 12,8 bis 12,9 eingestellt. Anschließend wird der pH-Wert durch Zugabe von 25ml 2N HCl auf 2,5 eingestellt und es wird mit 200ml Methylenchlorid extrahiert. Dir organische Phase wird mit 30ml Dimethylacetamid versetzt und die flüchtigen Anteile der Lösung werden im Vakuum bei <35°C abgedampft.
Zu der so erhaltenen Lösung werden 8,80g (*4R,5S,6S*)-(*p*-Nitrobenzyl) 3-[diphenylphosphonoloxy]-6-[(*1R*)-1-hydroxyethyl]-4-methyl-7-oxo-1-azabicyclo[3.2.0]hept-2-ene-2-carboxylat und 50ml Dimethylacetamid zugegeben und es wird auf -20°C temperiert. 4,26ml Tetramethylguanidin werden langsam zudosiert und es wird 30 Minuten nachgerührt. Danach wird bei 0°C auf eine Mischung von 4,0ml 85%iger *ortho*-Phosphorsäure, 100ml Wasser und 100ml Ethylacetat gegossen. Die organische Phase wird abgetrennt und 2 x mit je 50ml einer 10%igen NaCl Lösung in Wasser nachextrahiert und anschließend vorsichtig im Vakuum bei eingedampft.
Der Eindampfrückstand wird mit 50ml Dimethylacetamid verdünnt und auf -20°C gekühlt. Anschließend werden 3,72ml N,N-Diisopropylethylamin , gefolgt von 2,92ml Diphenylphosphinsäurechlorid so zudosiert, dass die Innentemperatur <-20°C bleibt.
Die Reaktionsmischung wird bei unter -15°C langsam zu einer Suspension von 5,50g Dimethylamin.Hydrochlorid in 50ml Dimethylacetamid und 12,1ml N,N-Diisopropylethylamin zudosiert und es wird 30min bei dieser Temperatur nachgerührt.
50ml Ethylacetat und 50ml 10%ige NaCl-Lösung werden zugegeben, die Phasen getrennt und die organische Phase noch 2x mit je 50ml 10% NaCl-Lösung gewaschen. Die Ethylacetat Phase wird über Na₂SO₄ getrocknet, filtriert und im Vakuum auf ca. 40ml eingeengt. Anschließend wird angeimpft und über Nacht bei 2-4°C stehen gelassen. Der kristalline Niederschlag wird abfiltriert, mit kaltem Ethylacetat gewaschen und im Vakuum getrocknet.
Auswaage: 7,8g
Die physikalischen und spektroskopischen Daten sind identisch wie bei dem Produkt aus Beispiel 1b).

### Beispiel 4: Meropenem

1,0g der Verbindung aus Beispiel 1 werden in 60ml Tetrahydrofuran und 90ml Wasser gelöst, mit 1,0g 10%igem Pd auf Aktivkohle versetzt und bei Atmosphärendruck 3h hydriert. Anschließend werden 50ml Ethylacetat zugegeben. Es wird filtriert und die organische Phase abgetrennt. Die wässrige Phase wird einmal mit Ethylacetat gewaschen und vorsichtig auf 5ml eingeengt. Zur wässrigen Lösung werden 30ml Tetrahydrofuran zugetropft, es wird angeimpft und man läßt bei 0°C kristallisieren. Der kristalline Niederschlag wird abfiltriert, mit Tetrahydrofuran gewaschen und im Vakuum getrocknet.

Auswaage: 0,48g
¹H-nmr (D₂O) δ 1.21 (d,3H,J=7.1 Hz), 1.29(d, 3H,J=6.3Hz), 1.97(m,1H), 2.99(s,3H), 3.06(s,3H), 3.09(m,1H), 3.38(m,1 H), 3.46(m,2H), 3.77(dd,1H,J=11.9&6.2Hz), 4.05(m,1H), 4.23(m,2H), 4.82(1 H, Signal liegt teilweise unter D₂O Signal)

## Patentansprüche

1. Eine Verbindung der Formel 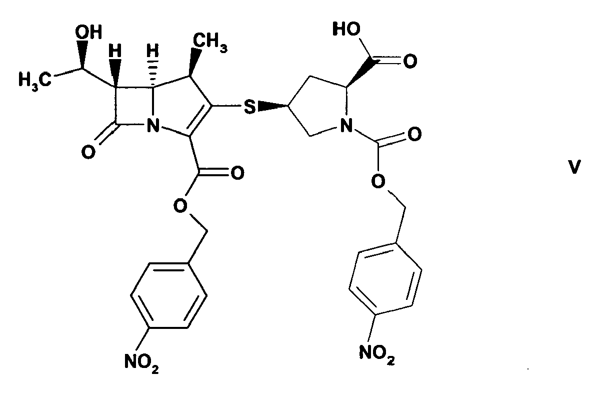 in freier Form oder in Form von Salzen.

2. Verbindung gemäss Anspruch 1 in Form eines Alkali- oder Erdalkalimetallsalzes oder in Form eines Ammonium-Salzes.

3. Verbindung gemäss Anspruch 2 in Form eines Salzes mit Kationen ausgewählt aus der Gruppe bestehend aus Natrium, Kalium, Lithium und Ammonium.

4. Verfahren zur Herstellung von Verbindungen der Formel V wie in Anspruch 1 definiert umfassend die Schritte
(i) Hydrolyse einer Verbindung der Formel 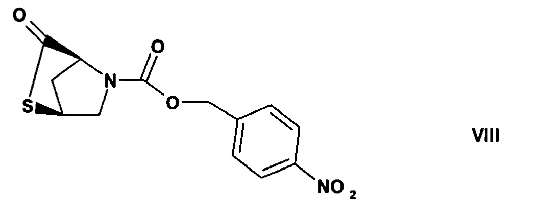 um eine Verbindung der Formel 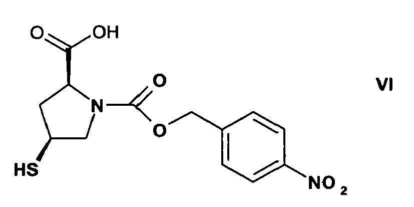 zu erhalten, und
(ii) Reaktion einer Verbindung der Formel 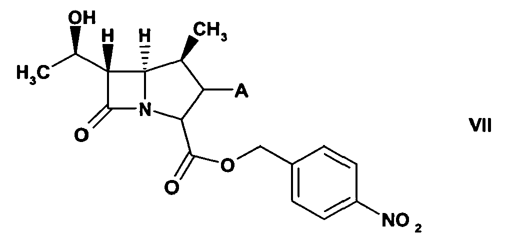
worin A für eine Aktivierungsgruppe steht, mit einer Verbindung der Formel VI wie aus Schritt (i) erhalten, um eine Verbindung der Formel V zu erhalten.

5. Verfahren gemäss Anspruch 4 wobei die Verbindung der Formel VI ohne Isolierung *in situ* in Schritt (ii) eingesetzt wird.

6. Verfahren für die Herstellung von 3-[*(3S,5S)*-5-carbamido-3-pyrrolidinyl]-thio substituierten Carbapenemen der Formel 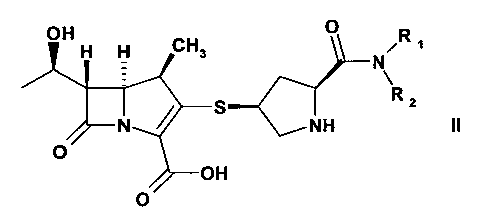 worin R₁ und R₂ unabhängig voneinander Alkyl, Wasserstoff, Aryl oder Heterocyclyl bedeuten,
oder R₁ und R₂ zusammen mit dem Stickstoffatom der Amid-Gruppe einen 5- bis 8 gliedrigen gesättigten oder ungesättigten Heterocyclus bilden, der unsubstituiert oder substituiert sein kann,
umfassend die Schritte
(A) Reaktion einer Verbindung der Formel V wie in Anspruch 1 definiert mit einem Amin der Formel 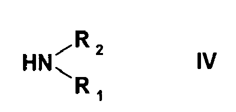 worin R₁ und R₂ dieselben Bedeutungen wie in Formel II haben, um eine Verbindung der Formel 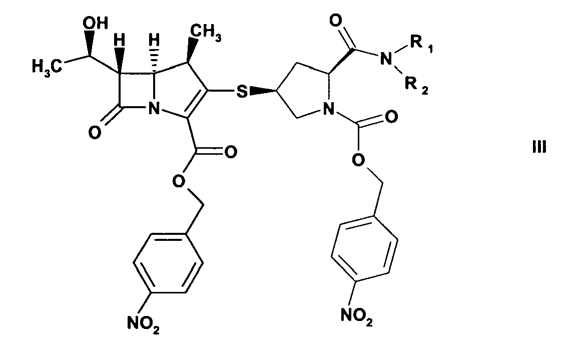 zu erhalten, worin R₁ und R₂ dieselben Bedeutungen haben wie oben für Formel II definiert,
(B) Abspaltung der p-Nitrobenzyl- bzw. p-Nitrobenzylcarbonyl-Schutzgruppen aus einer Verbindung der Formel III wie aus Schritt A erhalten, um eine Verbindung der Formel II zu erhalten.

7. Verfahren gemäss Anspruch 6 wobei R₁ und R₂ beide Methyl- bedeuten.

8. Verfahren gemäss Anspruch 6 wobei R₁ für Wasserstoff und R₂ für eine gegebenenfalls an der Carboxygruppe geschützte 3-Carboxyphenyl-gruppe stehen.

9. Verfahren gemäss Anspruch 6 wobei R₁ für Wasserstoff und R₂ für eine Gruppe 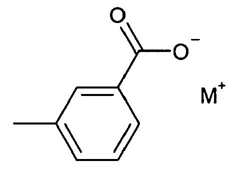 stehen, worin M⁺ ein Alkalimetall bedeutet.

10. Verwendung einer Verbindung der Formel V wie in Anspruch 1 definiert bei der Herstellung von 3-[(*3S,5S*)-5-carbamido-3-pyrrolidinyl]-thio substituierten Carbapenemen der Formel II wie in Anspruch 6 definiert.

11. Verwendung gemäss Anspruch 10 wobei das hergestellte Carbapenem Ertapenem oder Meropenem ist.

12. Verfahren zur Herstellung einer pharmazeutischen Zusammensetzung enthaltend ein Carbapenem der Formel II wie in Anspruch 6 definiert, umfassend die Schritte A und B wie in Anspruch 6 definiert und des weiteren umfassend das Mischen des aus Schritt B erhaltenen Verbindung der Formel II mit Lösungsmitteln oder Hilfsstoffen, um eine pharmazeutische Zusammensetzung enthaltend ein Carbapenem der Formel II zu erhalten.
